Europäisches Patentamt

European Patent Office

Office européen des brevets

(11) Veröffentlichungsnummer: **0 214 924 B1**

(12) **EUROPÄISCHE PATENTSCHRIFT**

(45) Veröffentlichungstag der Patentschrift: **26.08.92**

(21) Anmeldenummer: **86730118.6**

(22) Anmeldetag: **25.07.86**

(51) Int. Cl.⁵: **A61K 45/06**, A61K 37/34, A61K 31/565, //(A61K37/34, 31:565)

(54) **Oxytocin und Antigestagen zur Einleitung der Geburt.**

(30) Priorität: **05.09.85 DE 3531903**

(43) Veröffentlichungstag der Anmeldung:
**18.03.87 Patentblatt 87/12**

(45) Bekanntmachung des Hinweises auf die
Patenterteilung:
**26.08.92 Patentblatt 92/35**

(84) Benannte Vertragsstaaten:
**AT BE CH DE FR GB IT LI LU NL SE**

(56) Entgegenhaltungen:
**EP-A- 0 184 471**

**PATENT ABSTRACTS OF JAPAN, Band 1, Nr. 54, 25. Mai 1977, Seite 372 C 77; & JP-A-52 12 933 (KANEBO K.K.) 31-01-1977**

**CHEMICAL ABSTRACTS, Band 70, Nr. 15, 14. April 1969, Seite 50, Nr. 64762c, Columbus, Ohio, US; O. ZASZTOWT: "Effect of cortisol on the contractility of isolated ratuterus", & GINEKOL. POL. 1968, 39(10), 1093-101**

(73) Patentinhaber: **SCHERING AKTIENGESELL-SCHAFT Berlin und Bergkamen
Müllerstrasse 170/178 Postfach 65 03 11
W-1000 Berlin 65(DE)**

(72) Erfinder: **Chwalisz, Krzysztof, Dr.
Luzerner Strasse 1
W-1000 Berlin 45(DE)**
Erfinder: **Beier, Sybille, Dr.
Uhlandstrasse 121
W-1000 Berlin 31(DE)**
Erfinder: **Elger, Walter, Dr.
Schorlemerallee 12B
W-1000 Berlin 33(DE)**
Erfinder: **Neef, Günter, Dr.
Darmstädter Strasse 9
W-1000 Berlin 15(DE)**

**Beschreibung**

Die Erfindung betrifft die kombinierte Verwendung von Oxytocin bzw. (2-0-Methyltyrosin)-Oxytocin und einen Antigestagen für die Herstellung von Arzneimitteln zur Einleitung der Geburt. Oxytocin bzw. (2-0-Methyltyrosin)-Oxytocin und das Antigestagen finden dabei getrennte, vorzugsweise zeitlich abgestufte Anwendung bei der Einleitung der Geburt, wobei das Antigestagen vorzugsweise 12 bis 36 Stunden vor Oxytocin angewendet wird.

Oxytocin wird in der Geburtshilfe als wehenlösendes Mittel und zur Erhaltung der Uteruskontraktion während der Geburt verwendet. Die Dosierung erfolgt individuell durch i. m. oder s. c. Injektion, insbesondere durch i. v. Dauertropf oder auch durch buccale Anwendung.

Nachdem ursprünglich Präparate aus Hypophysenhinterlappen verwendet wurden, sind heute ausschließlich synthetische Präparate im Handel.

Ein Mittel zum Abbruch einer Schwangerschaft, das ein Antigestagen (Progesteronantagonisten) sowie eine uterotonisch wirksame Substanz, z. B. Oxytocin, enthält, ist bereits in der EP-A-0 184 471 beschrieben. Die Anwendung dieses Mittels bleibt auf eine frühe Phase der Schwangerschaft beschränkt.

Es ist bekannt, daß die Fähigkeit von Oxytocin, eine Wehentätigkeit des Uterus auszulösen, entscheidend von der Phase der Schwangerschaft abhängt. Erst gegen Ende der Gravidität besteht eine Oxytocinempfindlichkeit des Uterus, die einen Therapieversuch bei bestehender Notwendigkeit einer Geburtseinleitung aus fetaler oder mütterlicher Indikation aussichtsreich erscheinen läßt. Dennoch besteht auch am "Termin" das Problem einer großen Variabilität der Effekte einer Oxytocinbehandlung.

Bei der frühen Anwendung von Oxytocin, wenn die Zervix noch fest und geschlossen ist, beispielsweise nach vorzeitigem Blasensprung. kann es zu heftigen Kontraktionen des Uterus kommen, die sehr nachteilig für die Gesundheit des Kindes sein können.

Versuche mit der Kombination von Antigestagenen und Oxytocin führten nun zu der überraschenden Feststellung, daß die Oxytocinempfindlichkeit des Uterus durch Antigestagene entscheidend beeinflußt werden kann. Es wurde zudem beobachtet, daß es unter der Einwirkung des Antigestagens zu einer Erweichung und Erweiterung des Cervicalkanals kommt. Nach den vorliegenden Befunden können durch eine vorausgehende oder begleitende Antigestagenbehandlung in jeder Phase der Schwangerschaft Wehen durch Oxytocin ausgelöst und eine rasche Geburt erreicht werden. Man kann voraussagen, daß die vorgeschlagene Therapie die Dauer der Geburt erheblich verkürzt.

Nach einer bevorzugten Ausführungsform wird die Antigestagenbehandlung 12 bis 36 Stunden vor der Oxytocinbehandlung vorgenommen.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage:

$11\beta$-[(4-N,N-Dimethylamino)-phenyl]-$17\beta$-hydroxy-$17\alpha$-propinyl-4,9(10)-estradien-3-on,

$11\beta$-[(4-N,N-Dimethylamino)-phenyl]-$17\beta$-hydroxy-18-methyl-$17\alpha$-propinyl-4,9(10)-estradien-3-on und

$11\beta$-[(4-N,N-Dimethylamino)-phenyl]-$17a\beta$-hydroxy-$17a\alpha$-propinyl-D-homo-4,9(10)-16-estratrien-3-on (vgl. EP-A-0 057 115);

$11\beta$-Methoxyphenyl]-$17\beta$-hydroxy-$17\alpha$-ethinyl-4,9(10)-estradien-3-on [vgl. Steroids 37, (1981), 361 - 382],

$11\beta$-[(4-N,N-Dimethylamino)-phenyl]-$17\beta$-hydroxy-$17\alpha$-(hydroxyprop-1-(Z)-enyl)-4,9(10)-estradien-3-on (vgl. EP-A-0 147 361)

und insbesondere

$11\beta$-[(4-N,N-Dimethylamino)-phenyl]-$17\alpha$-hydroxy-$17\beta$-(3-hydroxypropyl)-$13\alpha$-methyl-4,9(10)-gonadien-3-on (vgl. EP-A-0 129 499).

Ebenfalls geeignet sind solche Antigestagene, die auf eine andere Weise als durch Kompetition am Gestagenrezeptor wirken. Beispielsweise genannt seien die Derivate des Epostans und Trilostans ($4\alpha,5\alpha$-Epoxy-3,$17\beta$-dihydroxy-$4\beta$,$17\alpha$-dimethyl-$5\alpha$-androst-2-en-2-carbonitril und $4\alpha,5\alpha$-Epoxy-3,$17\beta$-dihydroxy-$5\alpha$-androst-2-en-2-carbonitril, vgl. US-Patent 4,160,027), die die Synthese des Progesterons hemmen.

Die Antigestagene werden gemäß Erfindung in Mengen eingesetzt, die in der Regel unterhalb der sonst für den Schwangerschaftsabbruch üblichen Mengen liegen. Im allgemeinen wird man mit 10 - 200 mg $11\beta$-[(4-N,N-Dimethylamino)-phenyl]-$17\alpha$-hydroxy-$17\beta$-(3-hydroxypropyl)-$13\alpha$-methyl-4,9(10)-gonadien-3-on pro Tag oder einer physiologisch äquivalenten Menge eines anderen Antigestagens auskommen. Die Antigestagenbehandlung wird an 1 - 4, vorzugsweise 1 - 2 Tagen, vorgenommen.

Die Antigestagene können zum Beispiel lokal, topisch, enteral oder parenteral appliziert werden.

Für die bevorzugte orale Applikation kommen insbesondere Tabletten, Dragées, Kapseln, Pillen, Suspensionen oder Lösungen infrage, die in üblicher Weise mit den in der Galenik gebräuchlichen Zusätzen und Trägersubstanzen hergestellt werden können. Für die lokale oder topische Anwendung kommen beispielsweise Vaginalzäpfchen oder transdermale Systeme, wie Hautpflaster, infrage.

Eine Dosiseinheit enthält etwa 10 bis 200 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on oder eine biologisch äquivalente Menge eines anderen Antigestagens.

Die nachstehenden Beispiele sollen die galenische Formulierung von Antigestagenen erläutern.

Oxytocin oder Oxytocin-Analoga werden in den handelsüblichen Applikationsformen und Dosierungen angewendet. So enthalten Injektionslösungen pro ml 1 - 10 I.E. Oxytocin, Infusionslösungen pro 100 ml 0,5 - 2 I.E. Oxytocin und Buccaltabletten 100 - 300 I.E. Oxytocincitrat pro Tablette. Die für die künstliche Einleitung der Geburt wirksame Menge ist individuell verschieden; sie liegt in der Regel unterhalb der sonst üblichen Oxytocindosis und beträgt etwa 0,5 bis 5 I.E. Oxytocin in Form einer Infusions- oder Injektionslösung.

Beispiel 1

Zusammensetzung einer Tablette mit 10 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on zur oralen Applikation

```
  10,0 mg   11ß-/(4-N,N-Dimethylamino)-phenyl/-17d-hydroxy-
            17ß-(3-hydroxypropyl)-13d-methyl-4,9(10)-
            gonadien-3-on
 140,5 mg   Laktose
  69,5 mg   Maisstärke
   2,5 mg   Polyvinylpyrrolidon 25
   2,0 mg   Aerosil
   0,5 mg   Magnesiumstearat
 225,0 mg   Gesamtgewicht
 ========
```

Beispiel 2

Zusammensetzung einer öligen Lösung mit 50 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on zur parenteralen Applikation

In je 1 ml Rizinusöl/Benzylbenzoat im Volumenverhältnis 6 : 4 werden 50 mg des Antigestagens gelöst.

Pharmakologische Beobachtungen

Versuche:

Die Kombination von Antigestagen (AG) und Oxytocin wurde bei graviden Meeraschweinchen ca. 1 Woche vor dem natürlichen Geburtstermin untersucht:

1.) Antigestagen: 11$\beta$-[4-N,N-Dimethylamino)-phenyl]-17$\alpha$-hydroxy-17$\beta$-(3-hydroxypropyl)-13$\alpha$-methyl-4,9(10)-gonadien-3-on
Dosis/Applikation: 0,3 mg s.c.
Vehikel: 1 ml Benzylbenzoat/Rizinusöl ( 1 + 4)
Frequenz und Zeitpunkt der Behandlung:
Eine Injektion 18 Uhr, Tag 60 post coitum.
2.) Oxytocin (Syntocinon[R])
Dosis/Applikation: 25 mU/Tier/Injektion (1mU = 1 Tausendstel einer I.E.)
Vehikel: 1 ml, Handelsware verdünnt mit 0,9 % NaCl in aqua dest.

Frequenz und Zeitpunkt der Behandlung:

9 Uhr, Tag 61 post coitum,

Injektionen der o.g. Dosis alle 60 Minuten bis zur Geburt des ersten Foeten, maximal 6 Injektionen ( = 150 mU).

Gruppen

1.) Vehikelkontrollen
2.) Antigestagen allein
3.) Oxytocin allein
4.) Kombination Antigestagen/Oxytocin

Ergebnisse (s. Grafik)

Die Kombination von Antigestagen und Oxytocin führte nach wenigen Oxytocin-Injektionen zur Geburt bei allen behandelten 8 Muttertieren dieser Gruppe: Durchschnittliche Zahl der Oxytocin-Injektionen 3.7 ( = 93,75 mU Oxytocin), durchschnittliches Induktions-Geburtsintervall: 3.0 Stunden.

Andere Gruppen: Bei den Vehikelkontrollen wurden normale Geburten zum erwarteten Zeitpunkt beobachtet, d.h. nach Tag 65 post coitum. Oxytocin allein induzierte bei der applizierten Gesamtdosis von 150 mU gemäß Versuchsplan keine Geburt innerhalb der ersten 96 Stunden nach Beginn der Oxytocinbehandlung bei 5 Tieren dieser Gruppe. Die Behandlung mit 0,3 mg Antigestagen allein hatte ebenfalls keinen erkennbaren Effekt auf den Geburtsablauf und ging einher mit einem den Vehikel-Kontrollen vergleichbaren Geburtstermin, obwohl nach der Behandlung mit dem Antigestagen eine vorzeitig gereifte Zervix und ein für Oxytocin sensibilisiertes Myometrium vorgelegen haben dürfte.

Schlußfolgerung:

Die Kombination sonst völlig inaktiver Dosen von Antigestagen und Oxytocin führte zu einem voll wirksamen Verfahren zur Einleitung der Geburt durch eine präzise Aktivierung myometrialer Aktivität nach der Zervix-Reifung.

Die Effekte der getesteten Kombination eröffnen neue Perspektiven für die künstliche Einleitung der Geburt bei Tier und Mensch.

GEBURTSEINLEITUNG BEI MEERSCHWEINCHEN MIT ZEITLICH ABGESTUFTER AG/Ox–BEHANDLUNG:

0,3 mg AG, Tag 60 p.c., 18 Uhr, s.c.

25 m U/h Ox, Tag 61 p.c., 9 Uhr, s.c. (max. 150 mU)

**Patentansprüche**

1. Kombinierte Verwendung von Oxytocin bzw. (2-0-Methyltyrosin)-Oxytocin und einem Antigestagen für die Herstellung von Arzneimitteln zur Einleitung der Geburt.

**2.** Kombinierte Verwendung von Oxytocin bzw. (2-0-Methyltyrosin)-Oxytocin und einem Antigestagen gemäß Anspruch 1 in getrennten Dosiseinheiten.

**3.** Verwendung von Oxytocin gemäß Anspruch 1, dadurch gekennzeichnet, daß 1 bis 10 I. E. Oxytocin pro ml Injektionslösung, 0,5 bis 2 I. E. Oxytocin pro 100 ml Infusionslösung oder 100 bis 300 I. E. Oxytocin-citrat pro Buccaltablette verwendet werden.

**4.** Verwendung von 11$\beta$-(4-Dimethylaminophenyl)-17$\alpha$-hydroxy-17$\beta$-(3-hydroxypropyl)-13$\alpha$-methyl-4,9 (10)-gonadien-3-on gemäß Anspruch 1, dadurch gekennzeichnet, daß davon 10 bis 200 mg oder die physiologisch äquivalente Menge eines anderen Antigestagens verwendet werden.

## Claims

**1.** Combined use of oxytocin or (2-O-methyltyrosine)-oxytocin and an anti-gestagen for the preparation of medicaments for inducing labour.

**2.** Combined use of oxytocin or (2-O-methyltyrosine)-oxytocin and an anti-gestagen according to claim 1 in separate dosage units.

**3.** Use of oxytocin according to claim 1, characterised in that from 1 to 10 I.U. of oxytocin are used per ml of injection solution, from 0.5 to 2 I.U. per 100 ml of infusion solution or from 100 to 300 I.U. of oxytocin citrate per buccal tablet.

**4.** Use of 11$\beta$-(4-dimethylaminophenyl)-17$\alpha$-hydroxy-17$\beta$-(3-hydroxypropyl)-13$\alpha$-methyl-4,9(10)-gonadien-3-one according to claim 1, characterised in that from 10 to 200 mg of that compound or the physiologically equivalent amount of another anti-gestagen are used.

## Revendications

**1.** Utilisation combinée d'oxytocine ou de (2-O-méthyl-tyrosine-oxytocine et d'un antiprogestatif pour la fabrication de médicaments destinés à induire la naissance.

**2.** Utilisation combinée d'oxytocine ou de (2-O-méthyl-tyrosine)-oxytocine et d'un antiprogestatif selon la revendication 1 en des unités de prise séparées.

**3.** Utilisation d'oxytocine selon la revendication 1, caractérisée en ce qu'on utilise de 1 à 10 UI d'oxytocine par millilitre de solution injectable, de 0,5 à 2 UI d'oxytocine pour 100 ml de solution de perfusion, ou de 100 à 300 UI de citrate d'oxytocine par comprimé pour la voie buccale.

**4.** Utilisation de la 11$\beta$-(4-diméthylaminophényl)-17$\alpha$-hydroxy-17$\beta$-(3-hydroxypropyl)-13$\alpha$-méthyl-4,9(10)-gonadiène-3-one selon la revendication 1, caractérisée en ce qu'on utilise de 10 à 200 mg de ce composé ou la, quantité physiologiquement équivalente d'un autre antiprogestatif.